# EUROPEAN PATENT SPECIFICATION

(11) **EP 1 463 748 B1**
(45) Date of publication and mention of the grant of the patent: **24.10.2012**
(21) Application number: 03703702.5
(22) Date of filing: 07.01.2003
(51) Int. Cl.: C07J 31/00, A61K 31/566

(54) **SYNTHESIS OF A MIXTURE OF SULFATED ESTROGENS USING A SULFUR TRIOXIDE COMPLEX**
SYNTHESE EINER MISCHUNG VON SULFATIERTEN ÖSTROGENEN UNTER VERWENDUNG EINES SCHWEFELTRIOXIDKOMPLEXES
SYNTHESE D'UN MELANGE D'OESTROGENES SULFATES AU MOYEN D'UN COMPLEXE DE TRIOXYDE DE SOUFRE

(30) Priority: 08.01.2002 US 41916
(43) Date of publication of application: 06.10.2004
(73) Proprietor: Barr Laboratories, Inc., Woodcliff Lake, New Jersey 07677 (US)
(72) Inventor: LEONARD, Thomas, W., Wilmington, NC 28405 (US)
(74) Representative: Horner, Martin Grenville
(86) International application number: PCT/US2003/000275
(87) International publication number: WO 2003/057855

(56) References cited:
- US-A- 4 154 820
- US-A- 5 288 717
- US-A- 5 288 717
- US-A- 5 998 638
- US-A- 5 998 639
- US-A- 5 998 639
- US-B1- 6 458 778
- US-B1- 6 525 039

## Description

### Field of the Invention

The present invention relates to a process for the synthesis of a mixture of sulfated estrogens which may comprise sulfated Δ^{8,9}-dehydroestrone, estrone, equilin, and derivatives thereof, among others.

### Background

Naturally occurring estrogenic compositions are used in medical treatments to alleviate the symptoms of menopausal syndrome and osteoporosis/osteopenia in estrogen deficient women, prevent cardiovascular disease in men and women, and treat other hormone related disorders. The estrogenic components of the naturally occurring estrogenic compositions include sulfate esters of estrone, as disclosed in U.S. Patent No. 2,834,712.

The synthesis of sulfated estrogens has been described in past publications. For example, U.S. Patent No. 5,288,717 to Raveendranath et al. (Alkali Metal 8,9-Dehydroestrone Sulfate Esters) teaches a process of synthesizing alkali metal salts of 8,9-dehydroestrone (Δ^{8,9}-DHE) and its sulfate ester free from other conjugated esters present in material found in natural sources of mixed esters. In the process of Raveendranath, an alkali metal salt of Δ^{8,9}-DHE is initially produced followed by sulfation with sulfur trioxide-trimethylamine complex (SO₃-TMA) under mild conditions in an apolar, aprotic solvent such as tetrahydrofuran (THF) with simultaneous or subsequent addition of tris(hydroxymethyl)aminomethane (TRIS) as a stabilizer. The alkaline bases employed in the production of the initial intermediates of Δ^{8,9}-DHE are preferably sodium or potassium in the form of their hydrides and lithium as n-butyl lithium. This process provides a product free of other conjugated esters and does not teach production of several compounds at once.

U.S. Patent No. 5,998,639 to Raijmakers, et al. (Sulfatation of Estrogen Mixtures) teaches a process for the preparation of a mixture of sulphated estrogens containing Δ^{8,9}-DHE or derivatives thereof. In the process of Rajimakers, an estrogen mixture is obtained by isomerisation of equilin or a derivative thereof using lithium salts of ethylene diamine. The mixture is sulphated with sulphuric acid/acetic anhydride/pyridine. The mixture of crude pyridinesulfates is treated with sodium hydroxide in methanol, yielding a mixture in a specific ratio of Δ^{8,9}-DHE sodium sulphate and one or more of, for example, equilin sodium sulphate, 17α-dihydro equiline sodium sulphate, 17β-dihydro equilin sodium sulphate, 17α-estradiol sodium sulphate, and 17β-estradlol sodium sulphate.

There remains a need for an efficient process of producing a stable composition of a mixture of sulphated estrogens.

### Summary of the Invention

The present invention provides processes for the production of stable compositions comprising complex mixtures of sulphated estrogens. Previous synthetic procedures have involved synthesis of an estrogen, or a mixture of estrogens, from synthesis of a precursor. The present invention provides for the synthesis of complex estrogens by parallel synthetic processes on a mixture of precursors.
In particular, the present invention provides a process for the production of a stable composition comprising a mixture of sulphated estrogens, the process comprising the steps of:
a) reacting a sulphur trioxide complex with a mixture of alkali metals salts of estrogens to provide a mixture of sulphated alkali metal salts of estrogens; wherein the estrogens comprise at least two of Δ^{8,9}-dehydroestrone, estrone and equilin;
b) adding a stabilising amount of tris(hydroxymethyl)aminomethane; and
c) recovering the stable composition comprising the mixture of sulphated estrogens and tris(hydroxymethyl)aminometfiane, the recovered mixture of sulphated estrogens being in the same approximate ratios as that of the starting estrogens mixture.

The mixture of sulphated estrogens would correspondingly comprise sulphated alkali metal salts of Δ^{8,9}-DHE, estrone, equilin, or derivates thereof. These compounds are obtained in ratios not obtained when synthesized individually. The process comprises reacting a sulfur trioxide complex with a mixture of alkali metal salts of estrogens; adding a stabilizing amount of TRIS; and recovering the stable composition comprising the mixture of sulfated estrogens and TRIS. The process may further comprise reacting a mixture of estrogens with an alkali metal hydride to provide the mixture of alkali metal salts of estrogens. The process may be performed in an apolar, aprotic solvent. All steps of the process may also be performed in a single reaction vessel.

One advantage of the present invention is that the process produces a mixture of sulfated estrogens in a single vessel. In this respect, the mixture of alkali metal salts of Δ^{8,9}-DHE, estrone, equilin is sulfated simultaneously to provide a complex mixture of sulfated estrogen alkali metal salts having a potentially altered estrogenic composition and with ratios of the three primary estrogens that would not have been produced if synthesized individually. Furthermore, the entire reaction sequence of the process may be performed in a single vessel without isolating intermediate products.

In another aspect of the present invention, the process produces a mixture of sulfated estrogens in a specific ratio. By way of example, a mixture of estrogens comprising a specific ratio of Δ^{8,9}-DHE, estrone, equilin is provided. The process of the present invention is performed on this mixture of estrogens and produces a mixture of sulfated estrogens in the same approximate ratios as that of the starting estrogens.

### Detailed Description of the Illustrative Embodiments

According to the invention, a mixture of sulfated estrogens is produced, preferably using a single vessel. In the process of the present invention, a mixture of alkali metal salts of estrogens may be prepared from a first mixture of estrogens. Typically, the first mixture will contain at least two estrogens. The estrogens may be Δ^{8,9}-DHE, estrone, equilin.

The mixture of alkali metal salts of estrogens may be prepared by reacting the mixture of estrogens with an alkali metal hydride in an apolar, aprotic solvent. The mixture of alkali metal salts of estrogens may be sulfated using a sulfur trioxide complex in an apolar, aprotic solvent. For stability, an amount of TRIS may be added to the mixture of sulfated estrogens.

Accordingly, the general synthetic scheme as it applies to estrone and ^{Δ8,9}DHE is of the present invention is as follows:

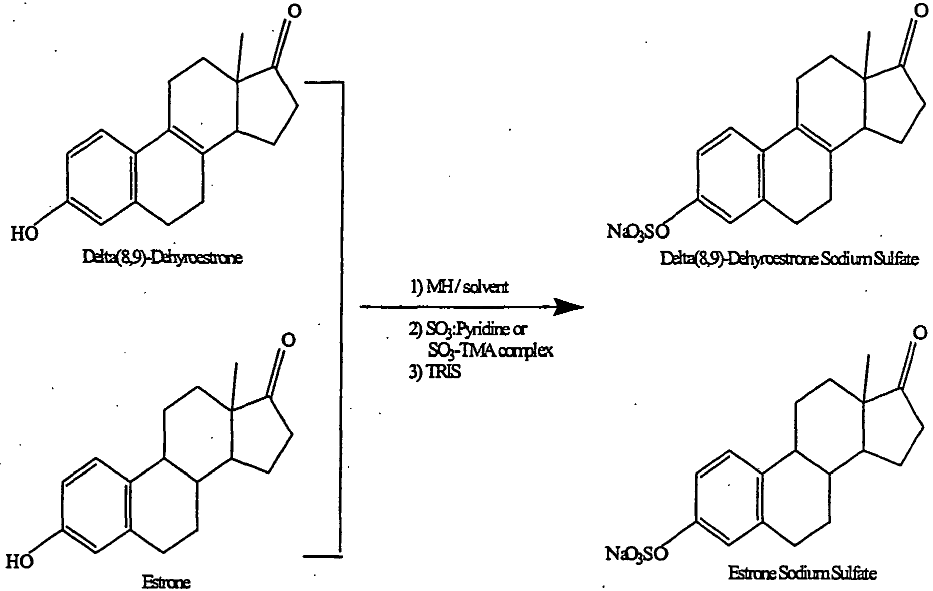

According to the general synthetic scheme of the present invention, a mixture of estrogens are reacted with an alkali metal hydride (MH), including for example, NaH, KH, LiH, and the like. This reaction may be performed in an apolar, aprotic solvent, including for example, THF, dioxane, diethyl ether, and the like. Where the mixture of estrogens comprises Δ^{8,9}-DHE, estrone this reaction produces a mixture of alkali metal salts of estrogens comprising alkali metal salts of Δ^{8,9}-DHE, estrone,

The mixture of alkali metal salts of estrogens is reacted with a sulfur trioxide complex, including for example, SO₃-TMA, SO₃-pyridine, and the like. This reaction also may be performed in an apolar, aprotic solvent, including, THF, dioxane, diethyl ether, and the like. Where the mixture of estrogens comprises Δ^{8,9}-DHE, estrone this reaction produces a mixture of sulfated alkali metal salts of estrogens comprising alkali metal salts of Δ^{8,9}-DHE, estrone.

To the mixture of sulfated estrogens is added a stabilizing amount of TRIS. These three reaction steps may be performed sequentially in a single reaction vessel without isolating the intermediate products. The composition comprising the mixture of sulfated estrogens and TRIS is recovered. As one of skill in the art would readily recognize, the composition may be recovered by any number of ways, including, for example, filtration, extraction, and the like. The resulting product may also be purified by any number of purification techniques, also well known in the art, including, for example, recrystallization, chromatography, and the like.

### Examples

The invention will be further explained by the following illustrative examples that are intended to be non-limiting.

### Example 1

Sodium hydride (NaH) (0.77 g, -0.0304 mole) and THF (60mL) under nitrogen atmosphere were added to a dry, 500 mL, three-neck, round bottom flask equipped with an air condenser, a 100 mL addition funnel, and magnetic stir bar. The suspension was stirred and cooled to 0-5°C. Next, Δ^{8,9}-DHE (5.11g, ∼0.0187 mole) dissolved in 75 mL THF, and solid estrone (1.27g, ∼0.0046 mole) were added at 0-5°C under a nitrogen atmosphere. After 30 minutes, the cooling bath was removed to allow the reaction mixture to attain ambient temperature, and the mixture was stirred for 2-2.5 hours at 20-22°C. Sulfur trioxide-pyridine complex (SO₃-Pyridine) (4.05g, ∼0.025 mole) was added in small batches to the reaction mixture. After stirring for 30 minutes, TRIS (2.82 g, ∼0.0233 mole) was added, and stirring was continued overnight at 20-22°C under a nitrogen atmosphere. The mixture was transferred into a 1 L round bottom flask. The solvent was evaporated under high vacuum (0.15 mm of Hg pressure) at 20°C, and then the pyridine was removed at 29-30°C. This step was repeated by adding 20 mL of fresh THF to the residue. The residue was taken up in 130 mL of deionized water, and the aqueous solution extracted nine times with 50 mL of diethyl ether to remove unreacted Δ^{8,9}-DHE and estrone. To the 130 mL of the aqueous solution were added 90 mL of deionized water and 1.6 g of activated carbon, "Darco." This was stirred for 30 minutes and filtered through a sintered glass funnel using filter agent "Celite-521." The carbon treatment was repeated by adding 1.6 g of "Darco," stirring for another 30 minutes, and filtering through a sintered glass funnel using filter agent "Celite-521." The TLC (thin-layer chromatography) of this solution [CHCl₃:MeOH:NH₄OH (25:5:1)] did not show a spot corresponding to Δ^{8,9}-DHE. The filtrate was lyophilized to obtain 9.1 g of light tan colored solid. The sample was analyzed by HPLC (high performance liquid chromatography), weight % process.

### Results

| | |
|---|---|
| Yield | = 9.1 g |
| HPLC Wt. % assay: DHES % | = 42.8% |
| ES % | = 12.2% |
| Total % HPLC wt. % | |
| Assay (DHES + ES) | = 55.0 |
| Molar Ratio of DHES:ES | = 1:0.29 |
| Molar Ratio of (DHES + ES):TRIS | = 1:1.73 |
| Moisture content by Karl-Fisher | = 3.2% |

### Example 2

The general process described in Example 1 was followed, except that SO₃-Pyridine was replaced by SO₃-TMA, and resultantly, the trimethylamine, as opposed to the pyridine, was removed.

Starting materials and reagents:

| Compound (purity) | Weight | Molar Amount |
|---|---|---|
| NaH (95%) | 0.64 g | ∼0.0253 mole |
| Δ^{8,9}-DHE (98%) | 5.02 g | ∼0.0183 mole |
| Estrone (99%) | 1.22 g | ∼0.0045 mole |
| SO₃-TMA (98%) | 3.66 g | ∼0.0258 mole |
| TRIS (99.9%) | 2.82 g | ∼0.0233 mole |

### Results

| | |
|---|---|
| Yield | = 8.1 g |
| HPLC Wt. % assay: DHES % | = 44.70% |
| ES % | = 9.54% |
| Total HPLC wt. % assay(DHES + ES) | = 54.24 |
| Molar Ratio of DHES:ES | = 1:0.21 |
| Molar Ratio of (DHES + ES):TRIS | = 1:1.96 |

### Example 3

The general process described in Example 2 was followed. Starting materials and reagents:

| Compound (purity) | Weight | Molar Amount |
|---|---|---|
| NaH (95%) | 2.01 g | ∼0.0793 mole |
| Δ^{8,9}-DHE (98%) | 10.0 g | ∼0.0366 mole |
| Estrone (99%) | 2.55 g | ∼0.0093 mole |
| SO₃-TMA (98%) | 11.5 g | ∼0.0811 mole |
| TRIS (99.9%) | 8.47 g | ∼0.0699 mole |

### Results

| | |
|---|---|
| Yield | = 23.79 g |
| HPLC Wt. % assay: DHES % | = 31.5% |
| ES % | = 9.1% |
| Total HPLC wt % assay(DHES + ES) | = 40.6 |
| Molar Ratio of DHES:ES | = 1:0.30 |
| Molar Ratio of (DHES + ES):TRIS | = 1:2.69 |

### Summary of Results

| | Molar | Ratios of | Starting | Materials | | | Results | | |
|---|---|---|---|---|---|---|---|---|---|
| | | | | | | | Molar Ratios | | |
| Exp. | DHE + E | NaH | SO₃-Amine | TRIS | DHES % | ES % | DHES:ES | (DHES + ES):TRIS | % Yield |
| 1 | 1 | 1.30 | 1.07 | 1.00 | 42.8% | 12.2 | 1:0.29 | 1:1.73 | 55.00 |
| 2 | 1 | 1.11 | 1.13 | 1.02 | 44.7% | 9.54 | 1:0.21 | 1:1.96 | 54.24 |
| 3 | 1 | 1.77 | 1.73 | 1.52 | 31.5% | 9.1 | 1:0.30 | 1:2.69 | 40.60 |

### Example 4

Chromatograms were compared to illustrate the differences between the processes taught herein and methods producing only one estrogenic compound at a time. Each chromatogram was obtained with the same chromatographic procedures. The chromatograms are detailed in Table 1 below which provides the counts per peak over time for the production of each of estrone, equilin, Δ^{8,9}-DHE (Delta-8,9), and a combination of these compounds produced by the processes of the invention (3-Combi). The table also provides ratios at each time indicated comparing each individual compound to the combination of estrogens prepared as taught herein. These relative ratios show the distinct differences in the results of the individual processes versus the combined process. As illustrated in the table, the processes of the invention enable the production of different ratios of estrogen products than can be obtained by preparing estrogenic compounds separately.

**Table 1**

| Time (min) | Counts/Peak | | | | Ratio | | |
|---|---|---|---|---|---|---|---|
| | Estrone | Equilin | Delta-8,9 | 3-Combi | Estrone: 3-Combi | Equilin: 3-Combi | Delta-8,9: 3-Combi |
| 25.721 | 0.50 | 0.50 | 0.50 | 3.05 | 0.16 | 0.16 | 0.16 |
| 24.984 | 0.50 | 0.50 | 0.50 | 6.15 | 0.08 | 0.08 | 0.08 |
| 22.152 | 0.50 | 53.81 | 0.50 | 139.62 | 0.00 | 0.39 | 0.00 |
| 21.975 | 0.50 | 0.50 | 0.50 | 32.07 | 0.02 | 0.02 | 0.02 |
| 21.634 | 0.50 | 0.50 | 0.50 | 4.33 | 0.12 | 0.12 | 0.12 |
| 20.237 | 0.50 | 0.50 | 204.47 | 41.58 | 0.01 | 0.01 | 4.92 |
| 19.436 | 0.50 | 0.50 | 10.50 | 31.66 | 0.02 | 0.02 | 0.33 |
| 18.722 | 4.08 | 0.50 | 0.50 | 31.13 | 0.13 | 0.02 | 0.02 |
| 18.376 | 0.50 | 0.50 | 15.00 | 74.69 | 0.01 | 0.01 | 0.20 |
| 15.818 | 0.50 | 15.51 | 1.00 | 3.27 | 0.15 | 4.74 | 0.31 |
| 12.591 | 0.50 | 0.50 | 3.91 | 18.93 | 0.03 | 0.03 | 0.21 |
| 11.944 | 0.50 | 0.50 | 15.75 | 2.78 | 0.18 | 0.18 | 5.67 |
| 10.981 | 0.50 | 0.50 | 0.50 | 4.22 | 0.12 | 0.12 | 0.12 |
| 10.373 | 0.50 | 0.50 | 0.50 | 2.68 | 0.19 | 0.19 | 0.19 |
| 10.222 | 0.50 | 0.50 | 0.50 | 2.18 | 0.23 | 0.23 | 0.23 |
| 9.910 | 0.50 | 0.50 | 8.57 | 3.33 | 0.15 | 0.15 | 2.58 |
| 8.901 | 0.50 | 0.50 | 0.50 | 4.01 | 0.12 | 0.12 | 0.12 |

## Claims

1. A process for the production of a stable composition comprising a mixture of sulphated estrogens, the process comprising the steps of:
a) reacting a sulphur trioxide complex with a mixture of alkali metal salts of estrogens to provide a mixture of sulphated alkali metal salts of estrogens; wherein the estrogens comprise at least two of Δ^{8,9}-dehydroestrone, estrone and equilin;
b) adding a stabilising amount of tris(hydroxymethyl)aminomethane; and
c) recovering the stable composition comprising the mixture of sulphated estrogens and tris(hydroxymethyl)aminomethane, the recovered mixture of sulphated estrogens being in the same approximate ratios as that of the starting estrogens mixture.

2. The process according to claim 1 wherein the sulphur trioxide complex is selected from the group consisting of sulphur trioxide-pyridine and sulphur trioxide-trimethylamine.

3. The process according to claim 1 wherein the alkali metal salt is selected from the group consisting of lithium, sodium and potassium.

4. The process according to claim 1 wherein steps a) and b) are performed in an apolar, aprotic solvent.

5. The process of claim 4 wherein the solvent is tetrahydrofuran.

6. The process according to claim 1 wherein all steps are performed in a single reaction vessel.

7. The process according to claim 1 further comprising the step of obtaining the mixture of alkali metal salts of estrogens by reacting a mixture of estrogens with an alkali metal hydride in an apolar, aprotic solvent.

8. The process according to claim 7 wherein the sulphur trioxide complex is selected from the group consisting of sulphur trioxide-pyridine and sulphur trioxide-trimethylamine.

9. The process according to claim 7 wherein the alkali metal salt is selected from the group consisting of lithium, sodium and potassium.

10. The process according to claim 7 wherein the apolar, aprotic solvent is tetrahydrofuran.

11. The process according to claim 7 wherein all steps are performed in a single reaction vessel.

12. A process according to claim 1 for the production of a stable composition comprising a mixture of sulphated estrogens, the process comprising the steps of:
a) reacting a mixture of estrogens with sodium hydride in an apolar, aprotic solvent to provide a mixture of alkali metal salts of the estrogens; herein the mixture of extrogens comprises at least two of Δ^{8,9}-dehydroestrone, estrone and equilin;
b) reacting sulphur trioxide-trimethylamine with the mixture of alkali metal salts of estrogens in an apolar, aprotic solvent to provide a mixture of sulphated alkali metal salts of estrogens;
c) adding a stabilising amount of tris(hydroxymethyl)aminomethane; and
d) recovering the stable composition comprising the mixture of sulphated estrogens and tris(hydroxymethyl)aminomethane, the recovered mixture of sulphated estrogens being in the same approximate ratios as that of the starting estrogens mixture.

13. The process according to claim 12 wherein the apolar, aprotic solvent is tetrahydrofuran.

14. The process according to claim 12 wherein all steps are performed in a single reaction vessel.

## Patentansprüche

1. Verfahren zum Herstellen einer stabilen Zusammensetzung, umfassend ein Gemisch aus sulfatierten Östrogenen, wobei das Verfahren die folgenden Schritte umfasst:
a) Reagieren eines Schwefeltrioxidkomplexes mit einem Gemisch aus Alkalimetallsalzen von Östrogenen, um ein Gemisch von sulfatierten Alkalimetallsalzen von Östrogenen bereitzustellen; wobei die Östrogene zumindest zwei von Δ^{8,9}-Dehydroöstron, Östron und Equilin umfassen;
b) Hinzufügen einer stabilisierenden Menge an Tris(hydroxymethyl)aminomethan; und
c) Gewinnen der stabilen Zusammensetzung, die das Gemisch von sulfatierten Östrogenen und Tris(hydroxymethyl)aminomethan umfasst, wobei das gewonnene Gemisch von sulfatierten Östrogenen in den ungefähr gleichen Verhältnissen wie das Ausgangs-Östrogengemisch vorliegt.

2. Verfahren nach Anspruch 1, wobei der Schwefeltrioxidkomplex aus der Gruppe bestehend aus Schwefeltrioxidpyridin und Schwefeltrioxidtrimethylamin ausgewählt ist.

3. Verfahren nach Anspruch 1, wobei das Alkalimetallsalz aus der Gruppe bestehend aus Lithium, Natrium und Kalium ausgewählt ist.

4. Verfahren nach Anspruch 1, wobei die Schritte a) und b) in einem apolaren aprotischen Lösungsmittel durchgeführt werden.

5. Verfahren nach Anspruch 4, wobei das Lösungsmittel Tetrahydrofuran ist.

6. Verfahren nach Anspruch 1, wobei alle Schritte in einem einzelnen Reaktionsgefäß durchgeführt werden.

7. Verfahren nach Anspruch 1, ferner umfassend den Schritt des Erhaltens des Gemischs von Alkalimetallsalzen von Östrogenen durch Reagieren eines Gemischs von Östrogenen mit einem Alkalimetallhyrid in einem apolaren aprotischen Lösungsmittel.

8. Verfahren nach Anspruch 7, wobei der Schwefeltrioxidkomplex aus der Gruppe bestehend aus Schwefeltrioxidpyridin und Schwefeltrioxidtrimethylamin ausgewählt ist.

9. Verfahren nach Anspruch 7, wobei das Alkalimetallsalz aus der Gruppe bestehend aus Lithium, Natrium und Kalium ausgewählt ist.

10. Verfahren nach Anspruch 7, wobei das apolare aprotische Lösungsmittel Tetrahydrofuran ist.

11. Verfahren nach Anspruch 7, wobei alle Schritte in einem einzelnen Reaktionsgefäß durchgeführt werden.

12. Verfahren nach Anspruch 1 zum Herstellen einer stabilen Zusammensetzung, die ein Gemisch aus sulfatierten Östrogenen umfasst, wobei das Verfahren die folgenden Schritte umfasst:
a) Reagieren eines Gemischs von Östrogenen mit Natriumhydrid in einem apolaren aprotischen Lösungsmittel, um ein Gemisch von Alkalimetallsalzen der Östrogene bereitzustellen; wobei das Gemisch von Östrogenen zumindest zwei von Δ^{8,9}-Dehydroöstron, Östron und Equilin umfasst;
b) Reagieren von Schwefeltrioxidtrimethylamin mit dem Gemisch von Alkalimetallsalzen von Östrogenen in einem apolaren aprotischen Lösungsmittel, um ein Gemisch von sulfatierten Alkalimetallsalzen von Östrogenen bereitzustellen;
c) Hinzufügen einer stabilisierenden Menge von Tris(hydroxymethyl)aminomethan; und
d) Gewinnen der stabilen Zusammensetzung, die das Gemisch von sulfatierten Östrogenen und Tris(hydroxymethyl)aminomethan umfasst, wobei das gewonnene Gemisch von sulfatierten Östrogenen in den ungefähr gleichen Verhältnissen wie das Ausgangs-Östrogengemischs vorliegt.

13. Verfahren nach Anspruch 12, wobei das apolare aprotische Lösungsmittel Tetrahydrofuran ist.

14. Verfahren nach Anspruch 12, wobei alle Schritte in einem einzelnen Reaktionsgefäß durchgeführt werden.

## Revendications

1. Procédé de fabrication d'une composition stable qui comprend un mélange d'oestrogènes sulfatés, qui comprend les étapes consistant à :
a) faire réagir un complexe de trioxyde de soufre avec un mélange de sels métalliques alcalins d'oestrogènes de façon à obtenir un mélange de sels métalliques alcalins d'oestrogènes sulfatés ; les oestrogènes comprenant au moins deux de Δ^{8,9}-déshydroestrone, d'estrone et d'équiline ;
b) l'ajout d'une quantité stabilisante de tris(hydroxyméthyl)aminométhane ; et
c) la récupération de la composition stable comprenant le mélange d'oestrogènes sulfatés et de tris(hydroxyméthyl)aminométhane, le mélange récupéré d'oestrogènes sulfatés étant dans les mêmes rapports approximatifs que ceux du mélange d'oestrogènes de départ.

2. Procédé selon la revendication 1, dans lequel le complexe de trioxyde de soufre est choisi parmi le groupe consistant en du trioxyde-pyridine de soufre et du trioxyde-triméthylamine de soufre.

3. Procédé selon la revendication 1, dans lequel le sel métallique alcalin est choisi parmi le groupe consistant en du lithium, du sodium et du potassium.

4. Procédé selon la revendication 1, dans lequel les étapes a) et b) sont réalisées dans un solvant apolaire et aprotique.

5. Procédé selon la revendication 4, dans lequel le solvant est du tétrahydrofurane.

6. Procédé selon la revendication 1, dans lequel toutes les étapes sont réalisées dans un réacteur unique.

7. Procédé selon la revendication 1, qui comprend en outre l'étape d'obtention du mélange de sels métalliques alcalins d'oestrogènes en faisant réagir un mélange d'oestrogènes avec un hydrure métallique alcalin dans un solvant apolaire et aprotique.

8. Procédé selon la revendication 7, dans lequel le complexe de trioxyde de soufre est choisi parmi le groupe consistant en du trioxyde-pyridine de soufre et du trioxyde-triméthylamine de soufre.

9. Procédé selon la revendication 7, dans lequel le sel métallique alcalin est choisi parmi le groupe consistant en du lithium, du sodium et du potassium.

10. Procédé selon la revendication, 7, dans lequel le solvant apolaire et aprotique est du tétrahydrofurane.

11. Procédé selon la revendication 7, dans lequel toutes les étapes sont réalisées dans un réacteur unique.

12. Procédé selon la revendication 1 pour la fabrication d'une composition stable qui comprend un mélange d'oestrogènes sulfatés, le procédé comprenant les étapes consistant à :
a) faire réagir un mélange d'oestrogènes avec de l'hydrure de sodium dans un solvant apolaire et aprotique afin d'obtenir un mélange de sels métalliques alcalins d'oestrogènes, le mélange d'oestrogènes comprenant au moins deux de Δ^{8,9}-déshydroestrone, d'estrone et d'équiline ;
b) faire réagir du trioxyde-triméthylamine de soufre avec le mélange de sels métalliques alcalins d' oestrogènes dans un solvant apolaire et aprotique afin d'obtenir un mélange de sels métalliques alcalins sulfatés d'oestrogènes ;
c) ajouter une quantité stabilisante de tris(hydroxyméthyl)aminométhane ; et
d) récupérer la composition stable comprenant le mélange d'oestrogènes sulfatés et de tris(hydroxyméthyl)aminométhane, le mélange récupéré d'oestrogènes sulfatés étant dans les mêmes rapports approximatifs que ceux du mélange d'oestrogènes de départ.

13. Procédé selon la revendication 12, dans lequel le solvant apolaire et aprotique est du tétrahydrofurane.

14. Procédé selon la revendication 12, dans lequel toutes les étapes sont réalisées dans un réacteur unique.
